# EUROPÄISCHE PATENTANMELDUNG

(11) **EP 2 724 671 A1**
(43) Veröffentlichungstag der Anmeldung: **30.04.2014**
(21) Anmeldenummer: 12189844.9
(22) Anmeldetag: 24.10.2012
(51) Int. Cl.: A61B 5/22, A61B 5/11, A23L 1/29

(54) **Verfahren zum Gewichtsmanagement einer Person**

(71) Anmelder: Weichselbaumer, Thomas Norbert, 4600 Wels (AT)
(72) Erfinder: Weichselbaumer, Thomas Norbert, 4600 Wels (AT)
(74) Vertreter: Jell, Friedrich

(57) **Zusammenfassung**

Es wird ein elektronisches Gerät und ein Verfahren zum Gewichtsmanagement einer Person, bei dem mithilfe eines Zeitmessgeräts Zeitdauern erfasst werden und in das Gewichtsmanagement eingehen, gezeigt. Um ein möglichst einfaches, aber dennoch effektives und individuell auf Personen abgestimmtes Verfahren zu ermöglichen, wird vorgeschlagen, dass wiederholt eine Karenzzeitdauer zwischen aufeinander folgenden, chemische Energie aufweisenden Nahrungsmittel- und/oder Getränkeaufnahmen der Person gemessen wird, wobei von den gemessenen Karenzzeitdauern jeweils ein von der körperlichen Aktivität beziehungsweise des Schlaf- oder Wachzustands der Person während dieser Karenzzeitdauer abhängiger Zeitwert abgezogen wird und die daraus errechneten Ergebnisse in das Gewichtsmanagement eingehen.

## Beschreibung

Die Erfindung betrifft ein Verfahren zum Gewichtsmanagement einer Person, bei dem mithilfe eines Zeitmessgeräts Zeitdauern erfasst werden und in das Gewichtsmanagement eingehen.

Aus dem Stand der Technik sind zahlreiche Verfahren zum Gewichtsmanagement (beispielsweise umfassend Maßnahmen zur Gewichtsreduktion, -zunahme und/oder -stabilisierung) einer Person bekannt, bei denen diverse Zeitdauern erfasst und berücksichtigt werden. Im Falle einer gewünschten Gewichtsreduktion wird dabei unter anderem versucht, möglichst lange Zeitdauern zwischen der Zufuhr chemische Energie aufweisender Lebensmittel oder Getränke - also möglichst lange Karenzzeitdauern - zu erreichen und die Zufuhr überschüssiger Kalorien zu vermeiden. Dazu wird unter anderem mithilfe unterschiedlichster Diätformen, aber auch durch Erhöhung der körperlichen Aktivität darauf geachtet, eine negative Kalorienbilanz zu erzielen - in der Annahme, dass dies den menschlichen Körper dazu veranlasst, das Energiedefizit aus seinem Fettgewebe zu decken. Auf diesen Prinzipien aufbauend werden unterschiedlichste Varianten zur Gewichtsreduktion angeboten, mit deren Hilfe vielfach auch Erfolg in Bezug auf eine Abnahme des Körpergewichts erzielbar ist, allerdings zeigt sich dieser nur selten langfristig in gewünschter Form. So beruht die Gewichtsreduktion nämlich oft zu einem hohen Anteil auf einem Abbau von Muskelmasse oder wird von einem derartigen Abbau begleitet, was nicht als vorteilhaft einzustufen ist. Des Weiteren stellen gängige Verfahren eine nicht unwesentliche psychische Belastung für die jeweilige Person dar, weshalb Verfahren des Stands der Technik vielfach frühzeitig abgebrochen werden.

Die Erfindung hat sich daher ausgehend vom Stand der Technik die Aufgabe gestellt, ein Verfahren zum Gewichtsmanagement einer Person zur Verfügung zu stellen, das die Stoffwechselprozesse des Körpers besser berücksichtigt, um dessen Effektivität zu verbessern und vor allem auch, um langfristige Erfolge zu gewährleisten.

Die Erfindung löst die gestellte Aufgabe dadurch, dass wiederholt eine Karenzzeitdauer zwischen aufeinander folgenden, chemische Energie aufweisenden Nahrungsmittel- und/oder Getränkeaufnahmen der Person gemessen wird, wobei von den gemessenen Karenzzeitdauern jeweils ein von der körperlichen Aktivität beziehungsweise des Schlaf- oder Wachzustands der Person während dieser Karenzzeitdauer abhängiger Zeitwert abgezogen wird und die daraus errechneten Ergebnisse in das Gewichtsmanagement eingehen.

Wird wiederholt eine Karenzzeitdauer zwischen aufeinander folgenden, chemische Energie aufweisenden Nahrungsmittel- und/oder Getränkeaufnahmen einer Person gemessen, kann zunächst unter anderem ihr Ernährungsverhalten überprüft und auch ausgewertet werden. Während der Karenzzeitdauer werden dem Körper demnach keine Kalorien in Form von Speisen oder Getränken zugeführt. Die Karenzzeitdauer endet mit jedweder Kalorienaufnahme in Form von Nahrungsmitteln oder Getränken. Auf Grundlage dieser Informationen und der entsprechenden Rückschlüsse können bestimmte Stoffwechselprozesse im Körper einer Person in Bezug auf das Gewichtsmanagement analysiert und je nach gewünschtem Effekt, beispielsweise Reduktion von Körperfett, gezielt genutzt werden. Erfindungsgemäß wird dazu vorgeschlagen, nun von den gemessenen Karenzzeitdauern jeweils einen von der körperlichen Aktivität beziehungsweise des Schlaf- oder Wachzustands der Person während dieser Karenzzeitdauer abhängigen Zeitwert abzuziehen. Insbesondere am Beispiel des Fettstoffwechsels, also des Auf- und Abbaus des Fettgewebes im Körper einer Person, wird das erfindungsgemäße Verfahren in weiterer Folge näher erläutert.

Bekanntermaßen erfolgt die Regulierung des Fettstoffwechsels durch das Zusammenspiel komplexer Mechanismen, etwa hormonell und enzymatisch. Im Wesentlichen kann jedoch gesagt werden, dass Insulin hierbei eine zentrale Rolle spielt. Daher sollen nachstehende Ausführungen der Einfachheit halber insbesondere in Bezug auf dieses Hormon erfolgen, wobei das Verfahren nicht ausschließlich als von Insulin abhängig oder auf diesem basierend zu verstehen ist. Generell kann davon ausgegangen werden, dass sich hohe Insulinspiegel hemmend auf den Vorgang der Lipolyse, also die Spaltung von Lipiden in Fettzellen des Fettgewebes und somit den Abbau von Körperfett, auswirken und den Aufbau von Fettgewebe fördern, während vergleichsweise niedrige Insulinspielgel im Blut die Lipolyse begünstigen. Da nach einer kohlenhydratreichen Mahlzeit der Insulinspiegel steigt, hat demnach eine gewisse Zeitspanne zu verstreichen, bis dieser auf ein derartiges Niveau abgefallen ist, dass ein Abbau von Lipiden in den Fettzellen des Fettgewebes gegenüber deren Aufbau überwiegen kann. Im Falle einer gewünschten Reduktion von Körperfett ist es also vorteilhaft, den Zeitpunkt dieser Umstellung zu kennen, um ihn gezielt nutzen zu können.

Dies erfolgt erfindungsgemäß, unter anderem mithilfe eines Zeitwerts, der als Zeitdauer definiert werden kann, die ab Beginn einer Karenzzeitdauer verstreicht, bis sich der Stoffwechsel einer Person auf einen vorwiegend Fett abbauenden umgestellt hat. In Bezug auf den Insulinspiegel kann der Zeitwert somit als jene Zeitdauer gesehen werden, in der die Lipolyse in den Zellen des Fettgewebes durch die postprandiale Insulinwirkung infolge einer sättigenden Mahlzeit am Beginn der Nahrungskarenz noch gehemmt ist. Wird ein derartiger Zeitwert von einer Karenzzeitdauer abgezogen, kann, natürlich nur, wenn die Karenzzeitdauer größer ist als der Zeitwert, zunächst eine Restzeitdauer der Karenzzeitdauer erhalten werden, während welcher eine Fett abbauende Stoffwechsellage überwiegt. Die daraus errechneten Ergebnisse können in das Gewichtsmanagement verbessert eingehen.

Ein bedeutender erfindungsgemäßer Vorteil ist, dass im Verfahren eine Abhängigkeit des Zeitwerts von der körperlichen Aktivität beziehungsweise vom Schlaf- oder Wachzustand der Person während der jeweiligen Karenzzeitdauer, von welcher dieser Zeitwert abgezogen wird, vorgesehen ist. Generell kann gesagt werden, dass dieser Zeitwert im Schlafzustand größer ist, als im Wachzustand, da sich der Energieverbrauch des Körpers im Wachzustand - beispielsweise tagsüber in der Arbeit - auf einem erhöhten Niveau befindet und aufgrund des dadurch rascher fallenden Blutzuckerspiegels auch der Insulinspiegel schneller absinkt. In dieser Weise wirkt sich auch körperliche Aktivität aus, weshalb sich der Zeitwert, der bei vergleichsweise hoher körperlicher Aktivität - beispielsweise gegenüber sitzender Bürotätigkeit - von der Karenzzeitdauer abgezogen wird, ebenfalls verringert, um eine sich früher einstellende, überwiegend Körperfett abbauende Stoffwechsellage zu berücksichtigen.

Im Zusammenhang mit körperlicher Aktivität kann das erfindungsgemäße Verfahren zahlreiche Aspekte vorteilhaft nutzen - und diese beim Gewichtsmanagement auch wesentlich gezielter berücksichtigen. Einerseits durch die, den Abbau von Körperfett direkt während der körperlichen Bewegung begünstigende Wirkung. Andererseits auch durch die, den Abbau von Körperfett unmittelbar im Anschluss an die körperliche Bewegung noch weiterhin begünstigende Wirkung, die solange anhält, bis nach der körperlichen Aktivität, dem Körper wieder Kalorien in Form von Speisen oder Getränken zugeführt werden. Daher kann im Verfahren auch vorgesehen sein, den Zeitraum zwischen dem Ende einer sportlichen Aktivität und der Uhrzeit der erstmals darauf folgenden Kalorienzufuhr zu erfassen und gezielt beim Gewichtsmanagement zu berücksichtigen. Dabei kann die körperliche Aktivität zusätzlich vorteilhaft vergleichsweise unabhängig von ihrer Art, Intensität etc. berücksichtigt werden.

Bekannt ist, dass erhöhte körperliche Aktivität durch Steigerung der Muskelmasse etc. auch den Grundumsatz erhöhen und so beispielsweise einen Abbau von Körperfett erleichtern kann.

So können also individuelle Unterschiede hinsichtlich des Übergangs in verschiedene Stoffwechsellagen erfasst und gesteuert werden. Dies erfolgt also wesentlich verbessert gegenüber dem Stand der Technik, in welchem primär die Auswirkung einer Fettverbrennung während sportlicher Betätigung unter genauen Vorgaben hinsichtlich erforderlicher Dauer, Intensität etc. Eingang findet - ohne jedoch den Einfluss von Sport in den darauffolgenden Stunden zu berücksichtigen und gezielt für das Verfahren zu nutzen.

Ebenso kann wiederum - vor allem mithilfe einer Beendigung einer Karenzzeitdauer - Berücksichtigung finden, dass körperliche oder sportliche Betätigung keinen oder nur geringen unmittelbaren Einfluss auf eine Körperfett abbauende Stoffwechsellage ausübt - etwa, weil die Person währenddessen ein kohlenhydrathaltiges Getränk konsumiert hat. In diesem Fall kann sich nämlich nur noch eingeschränkt und/oder nur bei erheblich verlängerter Ausübungsdauer eine vorwiegend fettabbauende Stoffwechsellage einstellen. Das Verfahren kann sich also durch seine verbesserte Berücksichtigung des Stoffwechsels - etwa im Gegensatz zur ausschließlichen Berücksichtigung eines Kalorienverbrauchs - auszeichnen.

Des Weiteren kann eine Person durch das Verfahren auch vorteilhaft dazu angeregt bzw. motiviert werden, ihre körperliche Aktivität über den Tag verteilt zu erhöhen, falls etwa ein Fettabbau gewünscht sein sollte. Dies kann die Nachhaltigkeit des Gewichtsmanagements erheblich verbessern.

Überraschend hat sich im erfindungsgemäßen Verfahren gezeigt, dass dieses komplexe Zusammenhänge im Körperstoffwechsel vergleichsweise einfach und trotzdem exakt nutzen kann. Zu berücksichtigen gilt zwar, dass dieser etwa entsprechend der Art einer körperlichen Aktivität, nach Geschlecht und Person individuell variiert, individuelle Abweichungen jedoch vergleichsweise gering sind. Trotzdem kann erfindungsgemäß natürlich vorgesehen sein, das Verfahren und insbesondere Zeitwerte durch Erfassung von Geschlecht, diverser körperlicher Zustände und/oder der körperlichen Leistungsfähigkeit, Ernährungsgewohnheiten, Muskelmasse etc. einer Person individuell zu bestimmen, festzulegen, regelmäßig an diesbezügliche Änderungen anzupassen und in das Verfahren einfließen zu lassen.

Vorteilhaft können durch das Verfahren aber auch länger andauernde Karenzzeitdauern, in denen im Körper beispielsweise verstärkt Muskulatur abbauende Prozesse in unerwünscht hohem Ausmaß erfolgen, vermieden werden.

Ein wesentlicher Vorteil dieses Verfahrens zum Gewichtsmanagement ist auch, dass dieses weitestgehend unabhängig von Lebensrhythmus oder auch Ernährungsverhalten und Essgewohnheiten einer Person durchgeführt werden kann. Es spielt also praktisch keine Rolle, zu welcher Tageszeit Mahlzeiten, Schlafphasen oder Wachphasen erfolgen, da die jeweilige Stoffwechsellage des Körpers einer Person mithilfe eines entsprechenden Zeitwerts berücksichtigt werden kann. Ebenso spielen die je nach Land, Nationalität etc. unterschiedlichen Ernährungsgewohnheiten von Personen nur eine untergeordnete Rolle, da das Verfahren gezielt bestimmte Stoffwechselfunktionen und deren Auswirkungen unabhängig von der Zusammensetzung und Art der Ernährung erfassen kann. Dadurch kann sich das Verfahren durch eine weitreichende Anwendbarkeit auszeichnen. Ebenso kann mithilfe des Verfahrens die Eigenverantwortung einer Person für ihr Gewichtsmanagement verbessert werden, da erfindungsgemäß auf einfache Weise die Abhängigkeit des Körperstoffwechsels von Zeitwerten durch die Definition von Karenzzeitdauern berücksichtigt wird. Ein langfristiger Erfolg kann dadurch erreicht werden.

Im Allgemeinen wird festgehalten, dass es natürlich im Rahmen der Erfindung liegt, auch ein Verfahren zum Gewichtsmanagement für Personen bereitzustellen, die Körpergewicht aufbauen möchten. Hierzu wird etwa darauf geachtet, dass deren Stoffwechsel in möglichst geringem Ausmaß in einen katabolen, Eiweiß oder Fett abbauenden, Zustand übergeht. In diesem Fall wird beim Gewichtsmanagement darauf geachtet, dass eine Person regelmäßig Mahlzeiten bzw. Energie haltige Getränke zuführt, und die Dauer einer eventuellen Nahrungskarenz, den von der körperlichen Aktivität und vom Schlaf- bzw. Wachzustand der Person abhängigen Zeitwert, nicht überschreitet. Das Verfahren kann sich also durch eine vielseitige Anwendbarkeit auszeichnen.

Wird als Beginn einer Karenzzeitdauer das Ende von Mahlzeiten nur dann herangezogen, wenn die Mahlzeiten eines Tages in Summe den täglichen, den Lebensumständen der Person entsprechenden Kalorienbedarf im Wesentlichen decken, kann sichergestellt werden, dass im Zuge eines Gewichtsmanagements eventuell nachteilige Stoffwechselvorgänge vermieden werden können. Es wird erwähnt, dass es im Zusammenhang mit der Deckung des Kalorienbedarfs keine Rolle spielt, wie viele Mahlzeiten eine Person dazu über einen Tag zu sich nimmt - somit kann ein individuelles Ernährungsverhalten verbessert berücksichtigt werden.

So kann ein wiederholtes und/oder ausgeprägtes Defizit in der Deckung des täglichen Kalorienbedarfs, das sich bekanntlich negativ auf das Gewichtsmanagement bzw. auch auf den körperlichen bzw. psychischen Zustand von Personen auswirken kann, verhindert werden. Insbesondere hat der in diesem Fall dann üblicherweise stattfindende Abbau von Muskelmasse zur Energiegewinnung Bedeutung, was sich wiederum nachteilig durch Verringerung des Grundumsatzes, Verschlechterung der körperlichen Leistungsfähigkeit und so weiter auswirkt.

Dadurch, dass eine das Verfahren ausführende Person gezielt dazu angehalten wird, darauf zu achten, ihren Kalorienbedarf im Wesentlichen zu decken, kann sich das erfindungsgemäße Verfahren gegenüber dem Stand der Technik also weiter auszeichnen - die Motivation der Person ein Gewichtsmanagement längerfristig zu betreiben, kann erhöht werden. Somit stellen sich Erfolge nachhaltig ein, wodurch sich das Verfahren gegenüber diversen Vorgaben zum Gewichtsmanagement, insbesondere natürlich von hypokalorischen Crash-Diäten, auszeichnen kann. Berücksichtigt ist im erfindungsgemäßen Verfahren auch, dass die Kalorienzufuhr durch die Mahlzeiten und Getränke eines Tages in Summe nicht über den täglichen, den individuellen Lebensumständen einer Person entsprechenden Kalorienbedarf hinausgeht. im Falle einer gewünschten Gewichtsreduktion ist dies eine grundlegende Voraussetzung.

Wird als Beginn einer Karenzzeitdauer ausschließlich das Ende einer Mahlzeit herangezogen, nach welcher die Person ein subjektiv ausreichendes Sättigungsgefühl aufweist, kann auf einfache Weise sichergestellt werden, dass eine ausreichende Nährstoffversorgung vorliegt und das erfindungsgemäße Verfahren unter gewünschten Voraussetzungen durchgeführt wird, wobei auch individuelle Voraussetzungen und Gewohnheiten einer Person verbessert berücksichtigt werden. Unter anderem spielt es nämlich keine Rolle, wie viele Mahlzeiten eine Person zu sich nimmt, bis diese ein entsprechendes Sättigungsgefühl aufweist - somit kann eine Karenzzeitdauer also auch nach der letzten von mehreren kleineren Mahlzeiten beginnen. Dies kann im Verfahren ebenfalls hinsichtlich des Erhalts von Muskelmasse, der Leistungsfähigkeit etc. genutzt werden. Des Weiteren kann auch vorteilhaft vermieden werden, dass sich - wie es im Falle einer gewünschten Gewichtsreduktion vorkommen kann - ein gestörtes Essverhalten oder sogar eine Essstörung entwickelt.

Wird der Zeitwert entweder als maximal 6 Stunden in der Wachphase der Person oder maximal 10 Stunden in dessen Schlafphase festgelegt, kann das Verfahren den Zeitpunkt einer Umstellung einer Stoffwechsellage in Abhängigkeit des Energieverbrauchs vereinfacht erfassen. Wie bereits ausgeführt, liegt in der Schlafphase ein niedrigerer Energieverbrauch vor, weshalb ein Insulinspiegel länger hoch und der Fettabbau längere Zeit erniedrigt ist. Diesem Umstand wird erfindungsgemäß Rechnung getragen. Des Weiteren kann vorgesehen werden, dass der Zeitwert individuell festgelegt wird - etwa verringert bei Personen, deren Körper einen erhöhten Grundumsatz aufweist oder die beruflich stärker gefordert sind. Es hat sich jedoch gezeigt, dass sich nach den angegebenen Maximalwerten bei praktisch allen Personen die angesprochene Umstellung des Stoffwechsels ergeben kann.

Berücksichtigt das Gewichtsmanagement eine ausschließliche Aufsummierung der errechneten Ergebnisse mit positiven Werten, dann werden Karenzzeitdauern mit vorwiegend Fett abbauenden Stoffwechsel auch dann nicht verringert, wenn ein oder mehrere Karenzzeitdauern kürzer als die entsprechenden Zeitwerte ausfallen. In weiterer Folge führen auch Tage, an denen das Gewichtsmanagement nicht in gewünschter Weise erfolgt, nicht zu einer Reduktion bereits aufsummierter Zeiträume, in denen es bereits zu einer Reduktion des Körperfettgewebes gekommen ist. So kann auch die Motivation einer Person, das Verfahren auszuführen, verbessert werden, da erreichte positive Ergebnisse erhalten bleiben.

Berücksichtigt das Gewichtsmanagement den Wert der täglichen Aufsummierung bis zu maximal 4 Stunden pro Tag und maximal 15 Stunden pro Woche, können Verbesserungen hinsichtlich der Motivation einer Person, das Verfahren durchzuführen, erreicht werden. Auf diese Weise kann verhindert werden, dass eine Person versucht, die Tage an denen die Lebensgewohnheiten nicht in der, im Verfahren gewünschten Weise erfolgen, durch Tage mit einer unvorteilhaft, übertrieben hohen Stundenzahl in der Aufsummierung zu kompensieren. Außerdem wird, da einer Person bekannt ist, dass über diese maximalen Werte hinaus keine zusätzliche Zeit mehr aufsummiert wird, wiederum ermöglicht, die Motivation zur Verbesserung der Lebensgewohnheiten im Falle einer gewünschten Körpergewichtsreduktion durch realistisch erreichbare Ziele zu steuern. So kann es verbessert gelingen, die Gefahr eines negativen, eventuell sogar gesundheitsschädlichen Verhaltens - etwa durch unerwünscht lange Karenzzeiten - zu vermeiden.

Ist die Aufnahme chemische Energie aufweisender Nahrungsmittel und/oder Getränke ab einer Karenzzeitdauer, die sich aus der Summe von Zeitwert dieser Karenzzeitdauer und 4 Stunden errechnet, vorgesehen, kann verbessert sichergestellt werden, dass die Gefahr des Abgleitens in eine erhöht Muskulatur abbauende Stoffwechsellage verbessert vermieden wird. So kann wie bereits ausgeführt erfindungsgemäß unter anderem erreicht werden, dass der Grundumsatz einer Person hoch bzw. die körperliche Leistungsfähigkeit erhalten bleiben und auch ein, etwa aufgrund erhöhter sportlicher Aktivität bedingter, Muskelaufbau nicht gehemmt wird.

Wird zu den errechneten Ergebnissen bis zu eine Stunde pro Tag hinzugerechnet, wenn von der Person an diesem Tag sportliche Aktivität im Ausmaß von mindestens 20 aufeinanderfolgenden Minuten erfolgt, nach welcher die Person das Gefühl von Muskelermüdung aufweist, kann eine gezielte Berücksichtigung eventuell durch Zeitwerte nicht erfasster Lebensgewohnheiten erfolgen. Es werden also Zeitdauern einer körperlichen Aktivität erfasst, summiert und im Verfahren berücksichtigt, wobei der Zeitpunkt der sportlichen Aktivität unerheblich ist. Ebenso kann die sportliche Aktivität vorteilhaft vergleichsweise unabhängig von ihrer Art berücksichtigt werden, als Ausdauer-, Kraftausdauer- und/oder Krafttraining, wichtig ist jedoch, dass die Intensität dieser Betätigung derart hoch ist, dass die Person danach das Gefühl von Muskelermüdung aufweist.

Sind bei der Karenzzeiterfassung Unterbrechungen vorgesehen, kann unter anderem ermöglicht werden, dass eine eventuelle Umstellung von Lebens- und/oder Ernährungsgewohnheiten einer Person in moderaterem Ausmaß erfolgt. Es hat sich gezeigt, dass das Verfahren wesentlich nachhaltigere Erfolge mit sich bringt, als durch radikale Umstellungen im Tagesablauf einer Person zu erzielen wären. In diesem Zusammenhang kann sich das erfindungsgemäße Verfahren gegenüber dem Stand der Technik auch dahingehend auszeichnen, dass Maßnahmen vorgesehen sind, mit deren Hilfe psychische Belastungen oder sogar zwanghaftes Verhalten, die letztendlich sogar in Essstörungen, Verlust von sozialen Kontakten oder dergleichen münden können, vermieden werden können. Besonders bewähren konnte sich eine Unterbrechung von 2 Tagen nach 5 aufeinanderfolgenden Tagen mit Nahrungskarenzerfassung.

Wird das Gewichtsmanagement entsprechend erfasster Parameter zu körperlichem und/oder psychischem Zustand der Person gesteuert, kann, wie bereits ausgeführt, auf unterschiedliche Bedingungen hinsichtlich Lebens- und Ernährungssituationen, körperlicher und geistiger Leistungsfähigkeit etc. eingegangen werden. Ebenso kann die Durchführung des Verfahrens auch unterschiedliche zu erreichende Ziele berücksichtigen - etwa hinsichtlich Abbau oder Erhalt von Körperfett. Dazu können beispielsweise Zeitwerte entsprechend festgelegt werden. Auch ist es möglich, das Verfahren auf einfache aber effektive Weise den Auswirkungen des Gewichtsmanagements oder Änderungen hinsichtlich der körperlichen Aktivität im Tagesablauf einer Person entsprechend anzupassen. So kann es letztendlich auch gelingen, eine Person mithilfe des erfindungsgemäßen Verfahrens verbessert zu motivieren, indem es das Wohlbefinden der Person berücksichtigt, was letztendlich wieder einen langfristigen Erfolg des Gewichtsmanagement sicherstellen kann.

Die Erfindung umfasst zudem ein elektronisches Gerät zur Durchführung des Verfahrens mit einer Eingabevorrichtung, mit einer Anzeigevorrichtung, einer Zeitmesseinrichtung, mit einer Recheneinheit und mit einem mit der Recheneinheit verbundenem Speicher. Hierbei kann insbesondere ein mobiles Endgerät vorgesehen sein, das derart ausgeführt ist, dass es von einer Person am Körper getragen werden kann, vorzugsweise wie eine Uhr, um das Verfahren unkompliziert über einen gesamten Tag ausführen zu können. Dies ist auch insofern von Vorteil, weil eine Person im Laufe eines Tages über Status - etwa die Dauer einer aktuellen Karenzzeit - informiert wird oder eventuell auch positive Motivation durch das elektronische Gerät empfangen kann. Somit kann das Verfahren von einer Person also unabhängig ausgeführt werden. Dies kann ebenso dazu beitragen, die Eigenverantwortung einer Person im Hinblick auf das Gewichtsmanagement zu stärken. Des Weiteren können mithilfe eines Speichers Daten auch längerfristig erfasst bleiben, ausgewertet und in weiterer Folge das Verfahren den Daten entsprechend adaptiert werden.

Das erfindungsgemäße Verfahren wird beispielsweise anhand von Ausführungsbeispielen näher erläutert.

Im Allgemeinen kann festgehalten werden, dass nur bei einem Stoffwechselzustand mit aktivierter Lipolyse in den Zellen des Körperfettgewebes die Voraussetzung zur Reduktion der Masse des Körperfettgewebes erfüllt ist, da die Zellen des Fettgewebes dann mehr Energie an das Blut zur Energieversorgung des Körpers abgeben, als sie aus dem Blut aufnehmen - ihre Masse reduziert sich dadurch.

Mithilfe eines Zeitmessgeräts werden Zeitdauern erfasst, die in das Gewichtsmanagement eingehen. Dabei wird wiederholt eine Karenzzeitdauer zwischen aufeinander folgenden, chemische Energie aufweisenden Nahrungsmittel- und/oder Getränkeaufnahmen der Person gemessen. Es ist also vorgesehen, dem Körper während einer Karenzzeitdauer keine Kalorien in Form von Speisen oder Getränken zuzuführen. Das Trinken von Wasser oder z.B. ungezuckertem Tee kann jedoch auch in der Karenzzeitdauer jederzeit erfolgen, ohne diese zu beenden, da darin keine Kalorien enthalten sind.

Von den gemessenen Karenzzeitdauern wird jeweils ein von der körperlichen Aktivität beziehungsweise des Schlaf- oder Wachzustands der Person während dieser Karenzzeitdauer abhängiger Zeitwert abgezogen. Die daraus errechneten Ergebnisse gehen in das Gewichtsmanagement ein. Dazu nutzt die Person ein mobiles Endgerät mit einer Eingabevorrichtung, mit einer Anzeigevorrichtung, mit einer Zeitmesseinrichtung, mit einer Recheneinheit und mit einem mit der Recheneinheit verbundenen Speicher.

In weiterer Folge wird das Verfahren vor allem in Bezug einer gewünschte Körperfettreduktion darlegt. Der Vollständigkeit halber wird erwähnt, dass im Falle einer gewünschten Körpergewichtszunahme die Zeiträume mit aktivierter Lipolyse in den Zellen des Fettgewebes mithilfe des erfindungsgemäßen Verfahrens gezielt vermieden werden können.

Beispiel 1: Eine Person, beendet um 7:00 Uhr eine Mahlzeit, nach welcher diese Person ein subjektiv ausreichendes Sättigungsgefühl aufweist. Letzteres ist Voraussetzung für den Beginn einer Karenzzeitdauer, deren Uhrzeit über die Eingabevorrichtung erfasst wird. Dabei spielt es keine Rolle, wie viele - etwa kleinere - Mahlzeiten erforderlich waren, um diese, ein subjektives Sättigungsgefühl verursachende Mahlzeit zum Beginn einer Karenzzeit zu erreichen. Über einen Zeitraum von 8 Stunden wird dem Körper anschließend keine Energie in Form von Kalorien haltigen Speisen oder Getränken zugeführt und keine mittelschwere Arbeit oder Schwerarbeit ausgeübt - und auch nicht geschlafen. Danach definiert die Person mit der Eingabevorrichtung 15:00 Uhr als das Ende der Karenzzeit, da diese um 15:00 Uhr wieder eine chemische Energie aufweisende Mahlzeit einnimmt.

In diesem Beispiel wird der Zeitraum von 2 Stunden mit aktivierter Lipolyse in den Zellen des Fettgewebes von 13:00 bis 15:00 Uhr, in dem es zu einer Reduktion der Masse des Körperfettgewebes kommt, durch die Recheneinheit des mobilen Endgerätes automatisch ermittelt, indem der Zeitwert von 6 Stunden von der gesamten Karenzzeitdauer von 8h subtrahiert wird, und die nach Verstreichen des Zeitwerts bis zum Ende der Karenzzeit verbleibende Zeit zur Steuerung des Gewichtsmanagements aufsummiert und gespeichert wird.

In der Wachphase der Person wird der Zeitwert als maximal 6 Stunden festgelegt. Den Grad ihrer körperlichen Aktivität kann diese Person mithilfe einer Eingabevorrichtung erfassen und den Zeitwert in der Wachphase somit durch Betätigung der Eingabevorrichtung auf die Intensität der körperlichen Belastung abstimmen. Da die Person in diesem Beispiel keine mittelschwere Arbeit oder Schwerarbeit ausübt, wird der Zeitwert, entsprechend Obenstehendem, mit dem maximalen Wert von 6 Stunden festgelegt. Der Zeitwert in der Wachphase würde sich bei vorwiegender mittelschwerer Arbeit während der Karenzzeitdauer auf 5 Stunden und bei vorwiegender Schwerarbeit während der Karenzzeitdauer auf 4 Stunden reduzieren.

Beispiel 2: Eine Person die keine mittelschwere Arbeit oder Schwerarbeit ausübt, beendet um 18:00 Uhr eine Mahlzeit, nach welcher diese Person ein subjektiv ausreichendes Sättigungsgefühl aufweist. Mit der Eingabevorrichtung wird diese Uhrzeit als Beginn der Karenzzeit definiert. Über einen Zeitraum von 13 Stunden wird dem Körper anschließend keine Energie in Form von Kalorien haltigen Speisen oder Getränken zugeführt. Die Person definiert mit der Eingabevorrichtung in diesem Fall 7:00 Uhr am nächsten Tag als das Ende der Karenzzeit, da um 7:00 Uhr wieder eine Mahlzeit eingenommen wird und bestätigt mit der Eingabevorrichtung, dass in der Karenzzeit zwischen 18:00 Uhr am Vortag und 7:00 Uhr am nächsten Tag die Schlafphase stattgefunden hat.

In diesem Beispiel wird der Zeitraum von 3 Stunden mit aktivierter Lipolyse in den Zellen des Fettgewebes von 4:00 bis 7:00 Uhr, in dem es zu einer Reduktion der Masse des Körperfettgewebes kommt, durch die Recheneinheit des mobilen Endgerätes automatisch ermittelt, indem der Zeitwert von 10 Stunden von der gesamten Karenzzeitdauer von 13h subtrahiert wird und die nach Verstreichen des Zeitwerts bis zum Ende der Karenzzeit verbleibende Zeit zur Steuerung des Gewichtsmanagements aufsummiert und gespeichert wird.

Während der Schlafphase werden die Glykogenspeicher des Körpers durch den geringeren Energieverbrauch im Vergleich zu einer Wachphase langsamer entleert. Dieser Umstand wird durch den, in der Schlafphase längeren Zeitwert berücksichtigt, indem dieser in der Schlafphase mit bis zu 10 Stunden festgelegt wird.

Die Zeitdauer von 2 Stunden (von 13:00 Uhr bis 15:00 Uhr) im Beispiel 1 und von 3 Stunden (von 4:00 Uhr bis 7:00 Uhr) im Beispiel 2 gehen in das Gewichtsmanagement ein. Sie werden über die Recheneinheit des mobilen Endgeräts ermittelt und in einem mit der Recheneinheit verbundenen Speicher erfasst. Des Weiteren erfolgt eine ausschließliche Aufsummierung der errechneten Ergebnisse mit positiven Werten. Es werden dabei also jene Zeiträume aufsummiert, in denen es durch aktivierte Lipolyse in den Zellen des Fettgewebes zu einer Reduktion der Masse des Körperfettgewebes kommt. Dies hat einen wesentlichen positiven Einfluss auf die Motivation der Person, das Verfahren längerfristig durchzuführen.

Im Falle einer gewünschten Körpergewichtsreduktion wird empfohlen, Zeiträume mit aktivierter Lipolyse in den Zellen des Fettgewebes, im Ausmaß von insgesamt 10 bis 15 Stunden pro Woche zu erreichen. Um durch das Verfahren keine radikale Umstellung der Lebens- und Ernährungsgewohnheiten zu verursachen, ist vorgesehen, die täglichen Aufsummierung bis zu maximal 4 Stunden pro Tag und maximal 15 Stunden pro Woche zu berücksichtigen. Über diese Maximalwerte wird die Person auch informiert, womit durch das Verfahren erfahrungsgemäß auch verbessert längerfristig Erfolge erzielt werden können.

Ist die Aufnahme chemische Energie aufweisender Nahrungsmittel und/oder Getränke ab einer Karenzzeitdauer, die sich aus der Summe von Zeitwert dieser Karenzzeitdauer und 4 Stunden errechnet, vorgesehen, kann vermieden werden, dass sich im Zuge des Verfahrens verstärkt nachteilige - vor allem Eiweiß abbauende - Stoffwechselzustände bei der Person einstellen. Ebenso sind, um unerwünschte Folgen einer zu radikalen Umstellungen der Lebens- und Ernährungsgewohnheiten, durch zu lange Zeiträume mit aktiver Lipolyse in den Zellen des Fettgewebes etc. zu vermeiden, bei der Karenzzeiterfassung Unterbrechungen, insbesondere von 2 Tagen nach 5 aufeinanderfolgenden Tagen mit Nahrungskarenzerfassung, vorgesehen. Hiervon wird die Person informiert.

Durch die Betätigung der Eingabevorrichtung des elektronischen Geräts wird auch die Uhrzeit der Durchführung sportlicher Aktivität erfasst werden. Es ist vorgesehen, dass zu den errechneten Ergebnissen bis zu eine Stunde pro Tag hinzugerechnet wird, wenn von der Person an diesem Tag sportliche Aktivität, insbesondere als Ausdauer-, Kraftausdauer- und/oder Krafttraining, im Ausmaß von mindestens 20 aufeinanderfolgenden Minuten erfolgt. Hat also eine Person sportliche Aktivität durchgeführt, die zu einer verspürten Muskelermüdung geführt hat - z.B. Muskelkräftigungsgymnastik - wird diese durch Aufsummierung mit Zeiträumen mit aktivierter Lipolyse in den Zellen des Fettgewebes entsprechend berücksichtigt. So kann beispielsweise nicht nur die Leerung der Glykogenspeicher berücksichtigt, sondern die Person auch zu Sport motiviert werden.

Wird das Gewichtsmanagement entsprechend erfasster Parameter zu körperlichem und/oder psychischem Zustand der Person gesteuert - hier ist beispielsweise eine Möglichkeit der Eingabe dieser Verfassungen vorstellbar - kann das Verfahren vorteilhaft auf jeweilige Entwicklung abgestimmt werden. So kann es unter anderem sinnvoll sein, Unterbrechungen länger vorzusehen, etwa falls die Person Motivationsprobleme aufweist.

Durch das erfindungsgemäße Verfahren erhält also eine Personen im Fall einer gewünschten Körpergewichtsreduktion auf praktikable Art und Weise Informationen darüber, wie lange die Voraussetzungen zur Reduktion des Körperfettgewebes bestanden haben. Sie erhält auch Kontrolle darüber, wie oft die Voraussetzungen zur Reduktion des Körperfettgewebes noch geplant und herbeigeführt werden müssen, um die empfohlenen 10 - 15 Stunden pro Woche, in denen es durch aktivierte Lipolyse in den Zellen des Fettgewebes zu einer Reduktion der Masse des Körperfettgewebes kommt, zu erreichen.

## Patentansprüche

1. Verfahren zum Gewichtsmanagement einer Person, bei dem mithilfe eines Zeitmessgeräts Zeitdauern erfasst werden und in das Gewichtsmanagement eingehen, **dadurch gekennzeichnet, dass** wiederholt eine Karenzzeitdauer zwischen aufeinander folgenden, chemische Energie aufweisenden Nahrungsmittel- und/oder Getränkeaufnahmen der Person gemessen wird, wobei von den gemessenen Karenzzeitdauern jeweils ein von der körperlichen Aktivität beziehungsweise des Schlaf- oder Wachzustands der Person während dieser Karenzzeitdauer abhängiger Zeitwert abgezogen wird und die daraus errechneten Ergebnisse in das Gewichtsmanagement eingehen.

2. Verfahren nach Anspruch 1, **dadurch gekennzeichnet, dass** als Beginn einer Karenzzeitdauer das Ende von Mahlzeiten nur dann herangezogen wird, wenn die Mahlzeiten eines Tages in Summe den täglichen, den Lebensumständen der Person entsprechenden Kalorienbedarf im Wesentlichen decken und nicht über diesen Kalorienbedarf hinausgehen.

3. Verfahren nach Anspruch 1 oder 2, **dadurch gekennzeichnet, dass** als Beginn einer Karenzzeitdauer ausschließlich das Ende einer Mahlzeit herangezogen wird, nach welcher die Person ein subjektiv ausreichendes Sättigungsgefühl aufweist.

4. Verfahren nach einem der Ansprüche 1 bis 3, **dadurch gekennzeichnet, dass** der Zeitwert entweder als maximal 6 Stunden in der Wachphase der Person oder maximal 10 Stunden in dessen Schlafphase festgelegt wird.

5. Verfahren nach einem der Ansprüche 1 bis 4, **dadurch gekennzeichnet, dass** das Gewichtsmanagement eine ausschließliche Aufsummierung der errechneten Ergebnisse mit positiven Werten berücksichtigt.

6. Verfahren nach einem der Ansprüche 1 bis 5, **dadurch gekennzeichnet, dass** das Gewichtsmanagement den Wert der täglichen Aufsummierung bis zu maximal 4 Stunden pro Tag und maximal 15 Stunden pro Woche berücksichtigt.

7. Verfahren nach einem der Ansprüche 1 bis 6, **dadurch gekennzeichnet, dass** die Aufnahme chemische Energie aufweisender Nahrungsmittel und/oder Getränke ab einer Karenzzeitdauer, die sich aus der Summe von Zeitwert dieser Karenzzeitdauer und 4 Stunden errechnet, vorgesehen ist.

8. Verfahren nach einem der Ansprüche 1 bis 7, **dadurch gekennzeichnet, dass** zu den errechneten Ergebnissen bis zu eine Stunde pro Tag hinzugerechnet wird, wenn von der Person an diesem Tag sportliche Aktivität, insbesondere als Ausdauer-, Kraftausdauer- und/oder Krafttraining, im Ausmaß von mindestens 20 aufeinanderfolgenden Minuten erfolgt, nach welcher die Person das Gefühl von Muskelermüdung aufweist.

9. Verfahren nach einem der Ansprüche 1 bis 8, **dadurch gekennzeichnet, dass** bei der Karenzzeiterfassung Unterbrechungen, insbesondere von 2 Tagen nach 5 aufeinanderfolgenden Tagen mit Nahrungskarenzerfassung, vorgesehen sind.

10. Verfahren nach einem der Ansprüche 1 bis 9, **dadurch gekennzeichnet, dass** das Gewichtsmanagement entsprechend erfasster Parameter zu körperlichem und/oder psychischem Zustand der Person gesteuert wird.

11. Elektronisches Gerät, insbesondere mobiles Endgerät, zur Durchführung des Verfahrens nach einem der Ansprüche 1 bis 10 mit einer Eingabevorrichtung, mit einer Anzeigevorrichtung, mit einer Zeitmesseinrichtung, mit einer Recheneinheit und mit einem mit der Recheneinheit verbundenen Speicher.
